# EUROPEAN PATENT SPECIFICATION

(11) **EP 1 985 609 B1**
(45) Date of publication and mention of the grant of the patent: **01.09.2010**
(21) Application number: 08153394.5
(22) Date of filing: 27.03.2008
(51) Int. Cl.: C07C 249/14

(54) **Method for recovering cyclohexanone oxime**
Verfahren zur Rückgewinnung von Cyclohexanonoxim
Procédé de récupération d'oxime de cyclohexanone

(30) Priority: 29.03.2007 JP 2007087734; 19.12.2007 JP 2007327759
(43) Date of publication of application: 29.10.2008
(73) Proprietor: Sumitomo Chemical Company, Limited, Tokyo 104-8260 (JP)
(72) Inventor: Yokota, Masashi, Ehime Ehime 2-6-326 (JP); Kuwahara, Kanji, Ehime Ehime 2-3-716 (JP)
(74) Representative: Vossius & Partner

(56) References cited:
- US-A- 4 141 896

## Description

The present invention relates to a process for recovering cyclohexanone oxime contained in the residue remaining after vaporizing a liquid cyclohexanone oxime by heating.

ε-caprolactam (hereinafter referred sometimes to as "lactam") is a monomer which is mainly used to synthesize nylon-6 used in the production of fibers and engineering plastics. As a process for producing lactam, a process of carrying out a vapor-phase Beckmann rearrangement reaction using a gaseous cyclohexanone oxime as a starting material is known.

In the vapor-phase Beckmann rearrangement reaction, first, a liquid cyclohexanone oxime is usually evaporated by heating using a wetted-wall evaporator to obtain a gaseous cyclohexanone oxime (refer to US2002/0143181A1). However, cyclohexanone oxime may cause thermal decomposition or thermal condensation during the step of vaporizing by heating to produce tar as a by-product since it is inferior in thermostability. It is necessary to remove tar out of the system in the form of a residue since it causes clogging of the wetted-wall evaporator. In this case, there arises a problem that the liquid cyclohexanone oxime is also removed out of the system together with tar and thus gaseous cyclohexanone oxime cannot be obtained in high yield.

US 4141896 refers to a process for the production of ε-caprolactam from cyclohexanone oxime by rearrangement in the gaseous phase.

Thus, an object of the present invention is to provide a process for efficiently recovering cyclohexanone oxime from the residue remaining after vaporizing the liquid cyclohexanone oxime by heating, using a simple and easy process, while taking account of the thermostability of cyclohexanone oxime.

Under these circumstances, the present invention provides a process for recovering cyclohexanone oxime, which comprises vaporizing a liquid cyclohexanone oxime by heating to obtain a cyclohexanone oxime gas (A1), distilling the remaining residue under a pressure lower than the pressure upon vaporization by heating, and recovering cyclohexanone oxime contained in the residue.

Furthermore, the present inventors have tested condensing a cyclohexanone oxime gas by a condenser so as to recover a cyclohexanone oxime gas separated by distillation under a low pressure in a liquid state in light of handiness in the case of reusing the recovered cyclohexanone oxime. However, they have found that when a cooling medium at a temperature of lower than 90°C is used, a crystal of cyclohexanone oxime may be deposited in a condenser thereby causing clogging in the condenser since cyclohexanone oxime has a high melting point of 90°C. Furthermore, when hot water at a temperature of 90°C or higher is used as a cooling medium of the condenser so as to prevent crystal deposition, the amount of uncondensed gas increases, resulting in non-negligible recovery loss since cyclohexanone oxime has a comparatively high vapor pressure. Also, crystal deposition in an effluent gas piping cannot be avoided, thereby causing clogging of the piping. As described above, in both cases, there was a problem that a continuous stable operation is obstructed by crystallization of cyclohexanone oxime. It is considered that the operation pressure upon distillation is controlled to normal pressure or higher so as to reduce the uncondensed gas when hot water at a temperature of 90°C or higher is used as the cooling medium of the condenser. In that case, production of the tar component as the by-product is promoted. The present inventors have intensively studied so as to solve these problems, thus providing the following inventions.
(1) A process for recovering cyclohexanone oxime, which comprises vaporizing a liquid cyclohexanone oxime by heating to obtain a cyclohexanone oxime gas (A1), distilling the remaining residue under a pressure lower than the pressure upon vaporization by heating, and recovering cyclohexanone oxime contained in the residue.
(2) The process for recovering cyclohexanone oxime according to paragraph (1), wherein the distillation is carried out under a pressure of 1.4 to 10 kPa (absolute pressure).
(3) The process for recovering cyclohexanone oxime according to paragraph (1) or (2), wherein a cyclohexanone oxime gas (A2) separated by distilling the residue is brought into contact with a solvent capable of dissolving cyclohexanone oxime to obtain a cyclohexanone oxime solution, and then cyclohexanone oxime is recovered.
(4) The process for recovering cyclohexanone oxime according to paragraph (1) or (2), wherein the cyclohexanone oxime gas (A2) separated by distilling the residue is condensed by a condenser in which a cooling medium at 90° C or higher circulates to obtain a cyclohexanone oxime condensate liquid (A3) and an uncondensed cyclohexanone oxime gas (A4) obtained simultaneously is brought into contact with a solvent capable of dissolving cyclohexanone oxime to obtain a cyclohexanone oxime solution, and then cyclohexanone oxime is recovered.
(5) The process for recovering cyclohexanone oxime according to paragraph (3) or (4), wherein the cyclohexanone oxime gas (A2) or the cyclohexanone oxime gas (A4) is brought into contact with the solvent on the surface of a cooling body.

The present invention exerts the effect of being capable of obtaining cyclohexanone oxime in a high recovery ratio, simply and easily, from a mixture of cyclohexanone oxime obtained as the residue after vaporizing the liquid cyclohexanone oxime and a tar component derived from cyclohexanone oxime. Moreover, according to the present invention, cyclohexanone oxime can be separated at a comparatively low temperature and also thermal degradation of the resulting cyclohexanone oxime can be suppressed since the residue is distilled under a low pressure. In addition, by bringing cyclohexanone oxime separated by distillation under a low pressure into contact with a solvent capable of dissolving cyclohexanone oxime to obtain a cyclohexanone oxime solution, it becomes possible to recover cyclohexanone oxime in a liquid state while suppressing agglomeration (crystal deposition) of cyclohexanone oxime in the condenser or the piping connected to the condenser, and thus handiness in the case of reusing the recovered cyclohexanone oxime can be improved.

By reusing the cyclohexanone oxime obtained by the present invention as a raw material for production of lactam through the vapor-phase Beckmann rearrangement reaction, the effect of being capable of establishing a production process of lactam which has high utilization efficiency of cyclohexanone oxime as a raw material and is excellent in productivity is also exerted.

Fig. 1 is a flow chart showing one embodiment of a process for recovering cyclohexanone oxime of the present invention.

Fig. 2 is a flow chart showing another embodiment of a process for recovering cyclohexanone oxime of the present invention.

Fig. 3 is a flow chart showing still another embodiment of a process for recovering cyclohexanone oxime of the present invention.

### Description of Reference Numerals

- 100:: Evaporator
- 101:: Vacuum distiller
- 102:: First stage condenser
- 103:: Gas-liquid contactor
- 104, 106:: Pump (vacuum pump)
- 105:: Cooling unit

The process for recovering cyclohexanone oxime of the present invention is a process for recovering cyclohexanone oxime from the residue remaining after vaporizing a liquid cyclohexanone oxime by heating so as to obtain a cyclohexanone oxime gas (A1) used as a raw material in the production of ε-caprolactam by a vapor-phase Beckmann rearrangement reaction. Specifically, the residue is a mixture of cyclohexanone oxime and a tar component composed of a thermally decomposed product of the cyclohexanone oxime, and the present invention pertains to a process for recovering cyclohexanone oxime by separating from the mixture.

Evaporation of the liquid cyclohexanone oxime by heating in the case of obtaining the residue is usually carried out in a state where the pressure is not reduced, namely, a state of normal pressure to an increased pressure. Specifically, evaporation is carried out by heating at a temperature of 130 to 170°C under a pressure of 101 to 140 kPa (absolute pressure).

In the present invention, the residue is distilled under a pressure which is lower than the pressure upon vaporization by heating for obtaining a cyclohexanone oxime gas (A1) (hereinafter referred sometimes as to "Low Pressure"). Since cyclohexanone oxime can be separated at a comparatively low temperature by distilling under Low Pressure, thermal degradation of the resulting cyclohexanone oxime can be suppressed, and thus a high recovery ratio can be achieved. For example, the distillation is preferably carried out under reduced pressure of 1.4 to 10 kPa (absolute pressure), and more preferably 1.4 to 8 kPa (absolute pressure). When the pressure upon distillation is too low, cyclohexanone oxime tends to be agglomerated. In contrast, when the pressure upon distillation is too high, thermal degradation of cyclohexanone oxime tends to proceed.

The temperature (distillation temperature) upon distillation is preferably from 90 to 135°C, and more preferably from 90 to 125°C. When the distillation temperature is lower than 90°C, cyclohexanone oxime tends to be agglomerated. In contrast, when the distillation temperature is too high, thermal degradation of cyclohexanone oxime tends to proceed.

The cyclohexanone oxime gas (A2) separated by distilling the residue under Low Pressure is (I) recovered as a cyclohexanone oxime solution by bringing into contact with a solvent capable of dissolving cyclohexanone oxime, or (II) condensed by a condenser in which a cooling medium at a temperature of 90°C or higher circulates to obtain a cyclohexanone oxime condensate liquid (A3), and then the uncondensed cyclohexanone oxime gas (A4) obtained simultaneously is preferably brought into contact with a solvent capable of dissolving cyclohexanone oxime to obtain a cyclohexanone oxime solution, which is then recovered. Consequently, handiness in the case of reusing the recovered cyclohexanone oxime can be improved while suppressing agglomeration (crystal deposition) of cyclohexanone oxime in the condenser or the piping connected thereto.

As the solvent, solvents capable of dissolving cyclohexanone oxime can be used. Specifically, alcohols such as methanol, ethanol, n-propanol, isopropanol, and t-butanol; toluene and cyclohexanone are preferably used.

When the cyclohexanone oxime gas (A2) or the cyclohexanone oxime gas (A4) is brought into contact with the solvent, either (i) a gas-liquid contactor which can bring the cyclohexanone oxime gas (A2) or (A4) as a vapor into contact with the solvent, or (ii) a condenser which can bring the cyclohexanone oxime gas (A2) or (A4) into contact with the solvent on the surface of a cooling body can be used. Specifically, the gas-liquid contactor (i) may be a contactor in which the solvent cooled to the temperature lower than a boiling point under an operation pressure of distillation under Low Pressure is sprayed in the vapor of the cyclohexanone oxime gas (A2) or (A4) using a spray nozzle and, for example, conventional gas absorption equipment such as a spray column and a packed column can be employed. In contrast, the condenser (ii) may be a condenser in which the solvent is sprayed over a condensate produced by cooling vapor of the cyclohexanone oxime gas (A2) or (A4) on the surface of a cooling body (heat exchanger, etc.) and, for example, conventional heat exchangers such as a multitubular heat exchanger can be employed.

By reusing the cyclohexanone oxime obtained by the present invention as a raw material for production of lactam through the vapor-phase Beckmann rearrangement reaction, it is possible to establish a production process of lactam which has high utilization efficiency of cyclohexanone oxime as a raw material and is excellent in productivity.

### EXAMPLES

The present invention will now be described in detail by way of Examples, but the present invention is not limited to the following Examples.

### Example 1

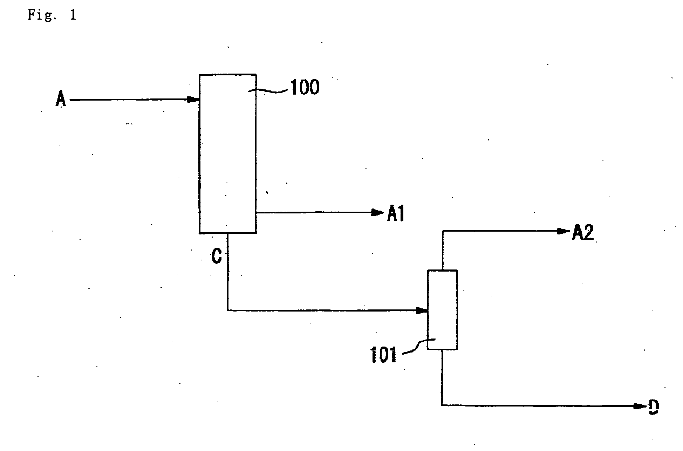

In accordance with the flow chart shown in Fig. 1, cyclohexanone oxime was recovered.

First, a liquid cyclohexanone oxime A was supplied to an evaporator 100 heated by steam (not shown) and then vaporized by heating under a pressure of 116 kPa (absolute pressure). As a result, a cyclohexanone oxime gas A1 was generated and also a mixture of cyclohexanone oxime and a tar component derived from cyclohexanone oxime was obtained as a residue C at a rate of 10 parts by weight/hour. The residue C was introduced into a vacuum distiller 101 heated by steam (not shown) and then distilled at 120°C after evacuating the distiller 101 to 4.9 kPa (absolute pressure) using a vacuum pump (not shown). Then, the cyclohexanone oxime A2 was separated at a rate of 9 parts by weight/hour and also a tar component D as a distilled residue was discharged from the bottom of the vacuum distiller 101 at a rate of 1 part by weight/hour.

### Example 2

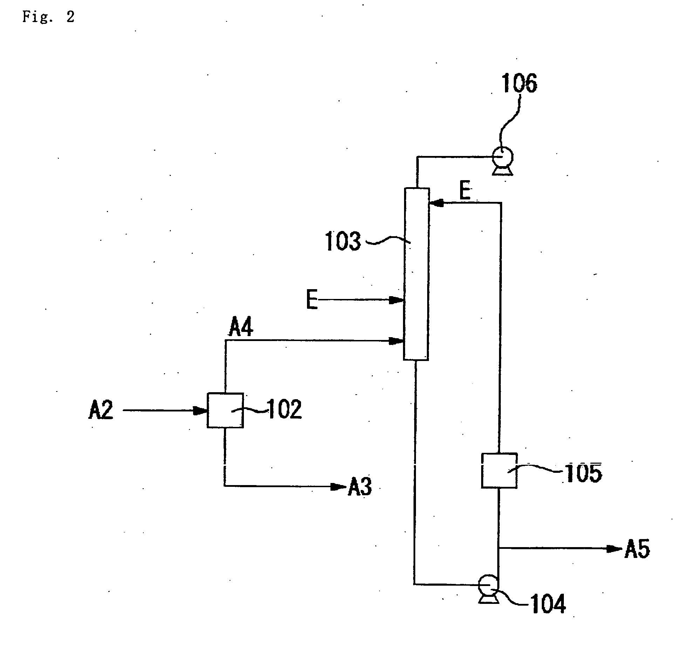

Using the cyclohexanone oxime A2 obtained by separating using the vacuum distiller 101 in Example 1, cyclohexanone oxime was recovered in accordance with the flow chart shown in Fig. 2.

The separated cyclohexanone oxime A2 was introduced into a first stage condenser 102 using hot water at 90°C and a liquid cyclohexanone oxime A3 condensed in the first stage condenser 102 was recovered, and also an uncondensed cyclohexanone oxime gas A4 was introduced into a gas-liquid contactor 103 while maintaining at 95°C. The gas-liquid contactor 103 is a packed column in which methanol E introduced continuously is circulated by a pump 104 and is sprayed through a spray nozzle (not shown) from the upper portion of the gas-liquid contactor 103, and then the cyclohexanone oxime gas A4 is liquefied by bringing methanol E cooled to -6°C using a cooling unit 105 directly into contact with the introduced cyclohexanone oxime gas A4 through spraying. Methanol containing the liquefied cyclohexanone oxime was extracted as a methanol solution A5 of cyclohexanone oxime during circulation. Cyclohexanone oximes A2 and A4 were introduced into the first stage condenser 102 and the gas-liquid contactor 103 using a vacuum pump 106.

### Example 3

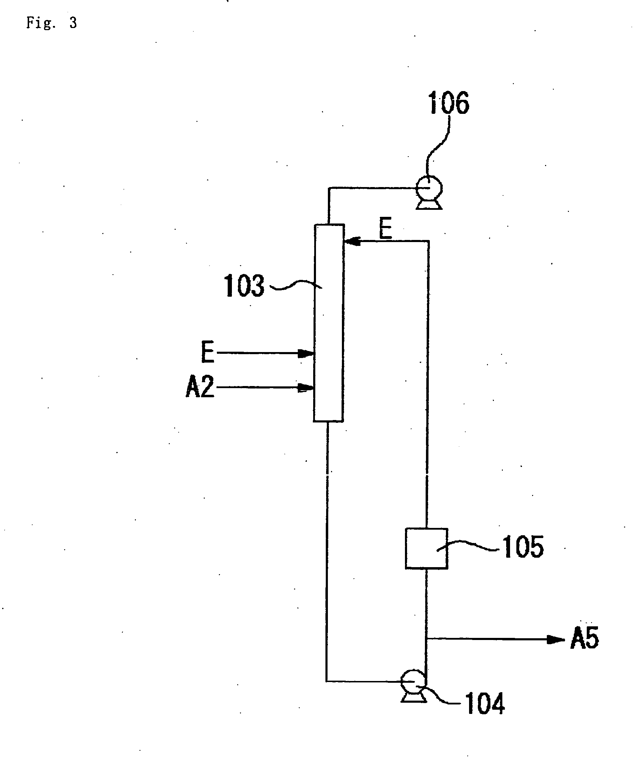

Using the cyclohexanone oxime A2 obtained by separating using the vacuum distiller 101 in Example 1, cyclohexanone oxime is recovered in accordance with the flow chart shown in Fig. 3.

The separated cyclohexanone oxime A2 was introduced into the gas-liquid contactor 103 using the vacuum pump 106. The gas-liquid contactor 103 is an equipment in which methanol E introduced continuously is circulated by the pump 104 and is sprayed through a spray nozzle (not shown) from the upper portion of the gas-liquid contactor 103, and then cyclohexanone oxime A2 is liquefied by bringing methanol E cooled to -6°C using the cooling unit 105 directly into contact with the introduced cyclohexanone oxime gas A2 through spraying. Methanol containing the liquefied cyclohexanone oxime can be extracted as a methanol solution A5 of cyclohexanone oxime during circulation.

The present invention exerts the effect of being capable of obtaining cyclohexanone oxime in a high recovery ratio, simply and easily, from a mixture of cyclohexanone oxime obtained as the residue after vaporizing the liquid cyclohexanone oxime and a tar component derived from cyclohexanone oxime. Moreover, according to the present invention, cyclohexanone oxime can be separated at a comparatively low temperature and also thermal degradation of the resulting cyclohexanone oxime can be suppressed since the residue is distilled under Low Pressure. In addition, by bringing cyclohexanone oxime separated by distillation under Low Pressure into contact with a solvent capable of dissolving cyclohexanone oxime to obtain a cyclohexanone oxime solution, it becomes possible to recover cyclohexanone oxime in a liquid state while suppressing agglomeration (crystal deposition) of cyclohexanone oxime in the condenser or the piping connected to the condenser, and thus handiness in the case of reusing the recovered cyclohexanone oxime can be improved.

By reusing the cyclohexanone oxime obtained by the present invention as a raw material for production of lactam through the vapor-phase Beckmann rearrangement reaction, the effect of being capable of establishing the production process of lactam which has high utilization efficiency of cyclohexanone oxime as a raw material and is excellent in productivity is also exerted.

## Claims

1. A process for recovering cyclohexanone oxime, which comprises vaporizing a liquid cyclohexanone oxime by heating to obtain a cyclohexanone oxime gas (A1), distilling the remaining residue under a pressure lower than the pressure upon vaporization by heating, and recovering cyclohexanone oxime contained in the residue.

2. The process for recovering cyclohexanone oxime according to claim 1, wherein the distillation is carried out under a pressure of 1.4 to 10 kPa (absolute pressure).

3. The process for recovering cyclohexanone oxime according to claim 1 or 2, wherein a cyclohexanone oxime gas (A2) separated by distilling the residue is brought into contact with a solvent capable of dissolving cyclohexanone oxime to obtain a cyclohexanone oxime solution, and then cyclohexanone oxime is recovered.

4. The process for recovering cyclohexanone oxime according to claim 1 or 2, wherein the cyclohexanone oxime gas (A2) separated by distilling the residue is condensed by a condenser in which a cooling medium at 90° C or higher circulates to obtain a cyclohexanone oxime condensate liquid (A3) and an uncondensed cyclohexanone oxime gas (A4) obtained simultaneously is brought into contact with a solvent capable of dissolving cyclohexanone oxime to obtain a cyclohexanone oxime solution, and then cyclohexanone oxime is recovered.

5. The process for recovering cyclohexanone oxime according to claim 3, wherein the cyclohexanone oxime gas (A2) or the cyclohexanone oxime gas (A4) is brought into contact with the solvent on the surface of a cooling body.

6. The process for recovering cyclohexanone oxime according to claim 4, wherein the cyclohexanone oxime gas (A2) or the cyclohexanone oxime gas (A4) is brought into contact with the solvent on the surface of a cooling body.

## Patentansprüche

1. Ein Verfahren zur Gewinnung von Cyclohexanonoxim, welches das Verdampfen eines flüssigen Cyclohexanonoxims durch Erwärmen, um ein Cyclohexanonoximgas (A1) zu erhalten, Destillieren des verbleibenden Rückstandes unter einem Druck, der niedriger ist als der Druck beim Verdampfen durch Erwärmen, und Gewinnen des in dem Rückstand enthaltenen Cyclohexanonoxims umfasst.

2. Das Verfahren zur Gewinnung von Cyclohexanonoxim nach Anspruch 1, wobei die Destillation unter einem Druck von 1,4 bis 10 kPa (absoluter Druck) durchgeführt wird.

3. Das Verfahren zur Gewinnung von Cyclohexanonoxim nach Anspruch 1 oder 2, wobei ein Cyclohexanonoximgas (A2), das durch Destillieren des Rückstandes abgetrennt wird, mit einem Lösungsmittel in Kontakt gebracht wird, das in der Lage ist, Cyclohexanonoxim zu lösen, um eine Cyclohexanonoximlösung zu erhalten, und dann Cyclohexanonoxim gewonnen wird.

4. Das Verfahren zur Gewinnung von Cyclohexanonoxim nach Anspruch 1 oder 2, wobei das Cyclohexanonoximgas (A2), das durch Destillieren des Rückstandes abgetrennt wird, durch einen Kondensator kondensiert wird, in welchem ein Kühlmedium bei 90°C oder mehr zirkuliert, um eine Cyclohexanonoxim-Kondensatflüssigkeit (A3) zu erhalten, und ein gleichzeitig erhaltenes nicht-kondensiertes Cyclohexanonoximgas (A4) mit einem Lösungsmittel in Kontakt gebracht wird, das in der Lage ist, Cyclohexanonoxim zu lösen, um eine Cyclohexanonoximlösung zu erhalten, und dann Cyclohexanonoxim gewonnen wird.

5. Das Verfahren zur Gewinnung von Cyclohexanonoxim nach Anspruch 3, wobei das Cyclohexanonoximgas (A2) oder das Cyclohexanonoximgas (A4) mit dem Lösungsmittel auf der Oberfläche eines Kühlkörpers in Kontakt gebracht wird.

6. Das Verfahren zur Gewinnung von Cyclohexanonoxim nach Anspruch 4, wobei das Cyclohexanonoximgas (A2) oder das Cyclohexanonoximgas (A4) mit dem Lösungsmittel auf der Oberfläche eines Kühlkörpers in Kontakt gebracht wird.

## Revendications

1. Procédé de récupération d'oxime de cyclohexanone, qui comprend la vaporisation d'un oxime de cyclohexanone liquide par chauffage pour obtenir un gaz d'oxime de cyclohexanone (A1), la distillation du résidu restant sous une pression plus basse que la pression de la vaporisation par chauffage ainsi que la récupération de l'oxime de cyclohexanone contenu dans le résidu.

2. Procédé de récupération d'oxime de cyclohexanone selon la revendication 1, dans lequel la distillation s'effectue à une pression comprise entre 1,4 et 10 kPa (pression absolue).

3. Procédé de récupération d'oxime de cyclohexanone selon la revendication 1 ou 2, dans lequel un gaz oxime de cyclohexanone (A2) séparé par distillation du résidu, est mis en contact avec un solvant capable de dissoudre l'oxime de cyclohexanone pour obtenir une solution d'oxime de cyclohexanone et ensuite on récupère l'oxime de cyclohexanone.

4. Procédé de récupération d'oxime de cyclohexanone selon la revendication 1 ou 2, dans lequel le gaz oxime de cyclohexanone (A2) séparé par distillation du résidu, est condensé par un condenseur dans lequel un fluide de refroidissement à 90°C ou plus circule pour obtenir un liquide de condensé d'oxime de cyclohexanone (A3) et un gaz oxime de cyclohexanone non condensé (A4) obtenu simultanément est mis en contact avec un solvant capable de dissoudre l'oxime de cyclohexanone pour obtenir une solution d'oxime de cyclohexanone, puis on récupère l'oxime de cyclohexanone.

5. Procédé de récupération d'oxime de cyclohexanone selon la revendication 3, dans lequel le gaz oxime de cyclohexanone (A2) ou le gaz oxime de cyclohexanone (A4) est mis en contact avec le solvant à la surface d'un corps de réfrigération.

6. Procédé de récupération d'oxime de cyclohexanone selon la revendication 4, dans lequel le gaz oxime de cyclohexanone (A2) ou le gaz oxime de cyclohexanone (A4) est mis en contact avec le solvant à la surface d'un corps de réfrigération.
